# EUROPEAN PATENT APPLICATION

(11) **EP 2 529 663 A1**
(43) Date of publication of application: **05.12.2012**
(21) Application number: 11188824.4
(22) Date of filing: 11.11.2011
(51) Int. Cl.: A61B 5/024

(54) **Method of diagnosing cardiopulmonary health through heart rate measurement and diagnostic system and recording medium therefor**

(30) Priority: 30.05.2011 KR 20110051038
(71) Applicant: Elancer, Inc., Seul 135-917 (KR)
(72) Inventor: Park, Woo Jin, 137-775 Seoul (KR); Ha, Yeon Tae, 305-503 Daejeon (KR)
(74) Representative: Hocking, Adrian Niall

(57) **Abstract**

Disclosed is a method of diagnosing cardiopulmonary health through a heart rate measurement and a diagnostic system and a recording medium Therefore, which can conveniently measure and analyze a heart rate and manage an electrocardiogram anytime and anywhere while overcoming time, space, and cost restraints, which are limits of a PAPS (Physical Activity Promotion System) performed under a situation where a certain environment and condition are constructed in order to measure and analyze a heart rate or a method of measuring and analyzing an electrocardiogram performed on a treadmill after a heart rate measurement sensor is attached to each part of a body of an athlete and the athlete wears a mask in order to measure a cardiopulmonary endurance of the athlete.

## Description

### BACKGROUND OF THE INVENTION

### 1. Field of the Invention

The present invention relates to a method of diagnosing cardiopulmonary health through a heart rate measurement, and a diagnostic system and a recording medium therefor. More particularly, the present invention relates to a method of diagnosing cardiopulmonary health through a heart rate measurement and a diagnostic system and a recording medium therefor, which can conveniently measure and analyze a heart rate and manage an electrocardiogram anytime and anywhere while overcoming time, space, and cost restraints, which are limits of a PAPS (Physical Activity Promotion System) performed under a situation where a certain environment and condition are constructed in order to measure and analyze a heart rate or a method of measuring and analyzing an electrocardiogram performed on a treadmill after a heart rate measurement sensor is attached to each part of a body of an athlete and the athlete wears a mask in order to measure a cardiopulmonary endurance of the athlete.

### 2. Description of the Related Art

In general, a cardiopulmonary endurance measurement has been performed using equipment located in a hospital or a specialized sports-related agency, and thus a general individual cannot test his/her own health of cardiopulmonary endurance or recognize a current level of his/her own cardiopulmonary endurance without specialized equipment.

Further, a method of measuring a cardiopulmonary endurance performed in the hospital or the specialized sports-related agency may be used in a limited space such as a treadmill since a heart rate measurement sensor, which is connected with an apparatus through a wired line, should be attached to each part of a body. Accordingly, the method cannot be used to measure and analyze a heart rate anytime and anywhere during a free exercise, and the method is cumbersome and costs are high since a lot of equipment should be used. The method of measuring the cardiopulmonary endurance is applied to a professional athlete, and it is difficult to apply the method to a general individual because of a high cost and a procedure complexity (a procedure of attaching a heart rate measurement sensor, a procedure of connecting a heart rate measurement sensor and a heart rate measurement apparatus, and a procedure of operating a heart rate measurement apparatus and analyzing a heart rate signal input to a heart rate measurement apparatus by a specialist).

A chest part of a body of a person is the best part to measure a heart rate while a heart rate measurement sensor is attached to the chest part because the chest part is less affected by a motion artifact. However, in a case of attaching the heart rate measurement sensor to the chest part, there is a cumbersomeness in that a person should take off his/her shirt and then put it on again and a problem in that in order to attach the heart rate measurement sensor to the chest part, an entire chest circumference should be compressed by a chest belt or the heart rate measurement sensor should be attached in a compressor type or with a tape.

Recently, various exercise applications using a smart phone have been greatly distributed so that a general athlete is assisted by the applications, but the exercise applications using the smart phone remain within a level where a service informing a user of an amount of burned calories and an exercise distance and route may be provided to the user by using an acceleration sensor and a Global Positioning System (GPS) installed in the smart phone. As such, there is no application for the smart phone which can provides information, which is actually required for an individual athlete for improving cardiopulmonary health after an exercise for a predetermined period.

### SUMMARY OF THE INVENTION

Accordingly, the present invention has been made to solve the above-stated problems occurring in the prior art, and an object of the present invention is to allow a heart rate to be measured and analyzed and an electrocardiogram to be managed conveniently anytime and anywhere.

In accordance with an aspect of the present invention, there is provided a system for diagnosing cardiopulmonary health by using a heart rate measurement, the system including: a heart rate measurement sensor for sensing a heart rate signal while being in contact with a temporal artery which is a connecting portion of an ear and a face of a user; a heart rate signal measurement device including an input unit for receiving an input of a heart rate signal measurement value from the heart rate measurement sensor being in contact with the temporal artery on a preset period, a wired/wireless communication unit for transmitting the heart rate signal measurement value to a reception means of the user, and a controller for controlling such that the heart rate signal measurement value through the heart rate measurement sensor is transmitted to the reception means of the user on the preset period by controlling the heart rate measurement sensor, the input unit, and the wired/wireless communication unit; a transmission means configured to have a body connecting an earphone, the heart rate measurement sensor, and the heart rate signal measurement device; a storing medium for storing information on an average recovery time (or recovery time slope) in accord with an age and a sex of the user, and a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise; a communication unit for receiving a heart rate signal measurement value transmitted from the transmission means; a determiner for analyzing a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise, comparing the analyzed recovery time (or recovery time slope) with the average recovery time (or recovery time slope) pre-stored in the storing medium, and then determining health of the cardiopulmonary function of the user; and the reception means configured to have an output unit for outputting a determination result of the determiner through a screen or a speaker.

According to the present invention, the method of diagnosing cardiopulmonary health through the heart rate measurement and the diagnostic system and the recording medium therefor having the above construction and operation provide an effect, in which a user can recognize his/her own heart rate health (cardiopulmonary endurance or electrocardiogram) anytime and anywhere without a separate cost generation and systematically manage an exercise effect after periodically exercising by using a heart rate measurement sensor, a reception means, and a recording medium analyzing a heart rate signal while being installed in the reception means.

### BRIEF DESCRIPTION OF THE DRAWINGS

The above and other aspects, features and advantages of the present invention will be more apparent from the following detailed description taken in conjunction with the accompanying drawings, in which:
FIG. 1 illustrates a position of a temporal artery (acupunctural region H14 or M3, 4);
FIG. 2 is an example of a heart rate measurement transmission means configured to measure a heart rate signal in a temporal artery;
FIG. 3 is a block diagram of a heart rate signal measurement apparatus of a system for diagnosing cardiopulmonary health through a heart rate measurement according to an embodiment of the present invention;
FIG. 4A is an example of a cardiopulmonary health diagnosis graph using a heart rate signal measured before/after exercise;
FIG. 4B is an example of a graph for a method of determining health of the cardiopulmonary function by using section recovery time 450, 455, and 460 and recovery time comparison straight line slopes 465, 470, and 475 for each section;
FIG. 4C is an example of a graph screen for displaying the exerciser recovery time slope 465 for each section or the exerciser recovery time slope 430 for entire section measured during a certain period together to manage a development of an improvement of health of the cardiopulmonary function according to exercise progress;
FIG. 5 is an example of a service construction for diagnosing health of the cardiopulmonary function by using a heart rate signal;
FIG. 6 is a block diagram illustrating an important construction of a smart phone which is a reception means of the present invention; and
FIG. 7 is a flowchart of a method of allowing the cardiopulmonary health of the exerciser to be identified anytime and anywhere by analyzing and displaying the measured heart rate signal.

### DETAILED DESCRIPTION OF THE EXEMPLARY EMBODIMENT

A system for diagnosing cardiopulmonary health by using a heart rate measurement, according to one embodiment of the present invention includes: a heart rate measurement sensor for sensing a heart rate signal while being in contact with a temporal artery which is a connecting portion of an ear and a face of a user; a heart rate signal measurement device including an input unit for receiving an input of a heart rate signal measurement value from the heart rate measurement sensor being in contact with the temporal artery on a preset period, a wired/wireless communication unit for transmitting the heart rate signal measurement value to a reception means of the user, and a controller for controlling such that the heart rate signal measurement value through the heart rate measurement sensor is transmitted to the reception means of the user on the preset period by controlling the heart rate measurement sensor, the input unit, and the wired/wireless communication unit; a transmission means configured to have a body connecting an earphone, the heart rate measurement sensor, and the heart rate signal measurement device; a storing medium for storing information on an average recovery time (or recovery time slope) in accord with an age and a sex of the user, and a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise; a communication unit for receiving a heart rate signal measurement value transmitted from the transmission means; a determiner for analyzing a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise, comparing the analyzed recovery time (or recovery time slope) with the average recovery time (or recovery time slope) pre-stored in the storing medium, and then determining health of the cardiopulmonary function of the user; and the reception means configured to have an output unit for outputting a determination result of the determiner through a screen or a speaker.

Here, the system further comprises: a storing medium for storing information on an average recovery time (or recovery time slope) in accord with an age and a sex of the user, and a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise; a communication unit for receiving a heart rate signal measurement value transmitted from the transmission means; a determiner for analyzing a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise, comparing the analyzed recovery time (or recovery time slope) with the average recovery time (or recovery time slope) pre-stored in the storing medium, and then determining health of the cardiopulmonary function of the user; and the reception means configured to have an output unit for outputting a determination result of the determiner through a screen or a speaker.

Here, the controller controls such that the heart rate measurement sensor measures a heart rate signal of the user on a preset period when the heart rate measurement sensor is a piezoelectric sensor, and supplies output power to a light emitting unit of a PPG sensor when the heart rate measurement sensor is the PPG sensor, wherein the controller controls such that a heart rate signal stronger than a predetermined intensity is measured by controlling an output of the power supplied to the light emitting unit of the PPG sensor through a control of a power unit when an intensity of the measured heart rate signal is weaker than a preset intensity.

Here, the controller further removes a motion artifact from a heart rate signal measurement value input through the input unit, and sets a period, on which a heart rate is measured, in the input unit.

Here, the determiner determines whether health of the cardiopulmonary function of the user is normal, good, or below average according to a size of a difference value between the analyzed recovery time (or recovery time slope) and the average recovery time (or recovery time slope) pre-stored in the storing medium.

Here, the determiner sets a step, in which an increased heart rate after a termination of an exercise is returned to a normal heart rate, for each section, to determine health of the cardiopulmonary function of the user for each section.

Here, the output unit outputs a recovery time (or recovery time slope) for an entire section or each section according to an exercise progress.

Here, the storing medium further stores information on a past average recovery time (or recovery time slope) of the user, and the determiner compares a recovery time (or recovery time slope) according to a current exercise by the user with the average recovery time (or recovery time slop) pre-stored in the storing medium, to determine health of the cardiopulmonary function of the user.

Here, the reception means transmits the determination result of the determiner to a third terminal or a server on a communication network.

Here, transmission means corresponds to a headset.

Here, the reception means corresponds to a smart phone.

In the meantime, a method of diagnosing cardiopulmonary health by using a heart rate measurement according to the present invention comprising: receiving a heart rate signal measurement value sensed by a heart rate measurement sensor for sensing a heart rate signal while being in contact with a temporal artery which is a connecting portion of an ear and a face of a user from a transmission means, by a reception means; generating a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise, by the reception means; comparing the generated recovery time (or recovery time slope) with a user's age average recovery time (or recovery time slope) or user's previous average recovery time (or recovery time slope) pre-stored in a storing medium to determine health of a cardiopulmonary function of the user, by the reception means; and outputting a comparison result between the generated recovery time (or recovery time slope) and the user's age average recovery time (or recovery time slope) or the user's previous average recovery time (or recovery time slope) pre-stored in the storing medium or a result of the determination on a screen, by the reception means.

Here, the comparison or the determination result is transmitted to a server on a communication network or a third terminal.

Hereinafter, a method of diagnosing cardiopulmonary health through a heart rate measurement and a diagnostic system and a recording medium therefor according to the present invention will be described in detail with reference to embodiments.

According to the present invention, a user can measure and analyze a cardiopulmonary endurance anytime and anywhere after an exercise or even during the exercise while overcoming space and time restraints of a method of measuring and analyzing a cardiopulmonary endurance performed by a hospital or a specialized sports-related agency. Further, a heart rate signal measured to determine whether current health of a cardiopulmonary function is good is transmitted to a reception means and then the reception means may analyze the received heart rate signal.

In addition, a part suitable for measuring a heart rate through a piezoelectric sensor or a PPG sensor while overcoming an effect of a motion artifact is a temporal artery (acupunctural region H14 or M3, 4), which is sunken part located in a central position where an ear and a face are connected to each other except for a chest of a body of a person. The temporal artery (acupunctural region H14 or M3, 4) is one of body parts, through which an accurate heart rate measurement may be performed, while overcoming an effect of a motion artifact generated during exercise because the temporal artery has a smaller movement in comparison with a wrist during exercise and through which a stronger heart rate signal than a heart rate signal measured in the wrist is transferred.

The present invention has a heart rate measurement sensor configured to be included in a headset which is a reception means generally used during daily life and exercise in order to attach the heart rate measurement sensor to the temporal artery (acupunctural region H14 or M3, 4), so that a user can measure and analyze an accurate heart rate during the exercise, after the exercise, or in daily life without separate additional accessories, labors, or costs.

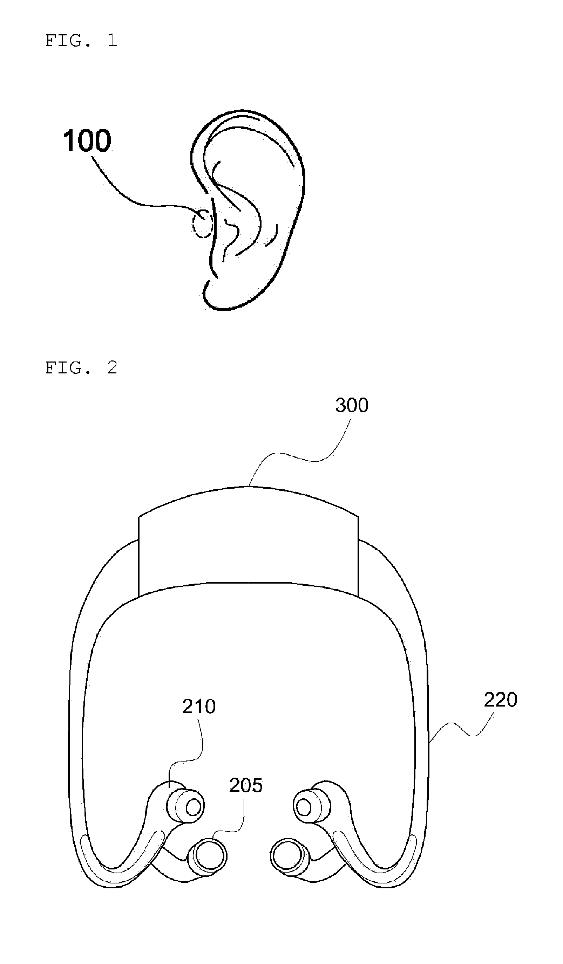

FIG. 1 is an example for a position of a temporal artery 100 to which a heart rate measurement sensor 205 is attached for measuring an accurate heart rate signal.

According to the present invention, the reason why the heart rate measurement sensor 205 is attached to the temporal artery 100 for measuring the heart rate signal is that the temporal artery (acupunctural region H14 or M3, 4) 100 is one of the body parts, through which an accurate heart rate measurement may be performed, while overcoming an effect of a motion artifact generated during exercise because the temporal artery has a smaller movement in comparison with a wrist during exercise and through which a stronger heart rate signal than a heart rate signal measured in the wrist is transferred. Measuring the heart rate signal in the temporal artery 100 is one of characteristics of the present invention in order to measure an accurate heart rate in a face part of the person.

FIG. 2 is an example of a heart rate measurement transmission means configured to measure a heart rate signal in a temporal artery.

FIG. 2 illustrates an example for measuring a heart rate signal in the temporal artery 100 by using a headset among transmission means frequently used during exercise, and the present invention is not limited by the example of FIG. 2.

The headset measuring a heart rate signal in the temporal artery 100 includes the PPG or piezoelectric-typed heart rate measurement sensor 205, an earphone 210 used for listening to music, a heart rate signal measurement device 300 controlling the heart rate measurement sensor 205 and the earphone 210, and a body 220 connecting the heart rate measurement sensor 205, the earphone 210 and heart rate signal measurement device 300 to each other.

In order to accurately place the heart rate measurement sensor 205 on the temporal artery 100 more closely, positions of the heart rate measurement sensor 205 and the earphone 210 may be exchanged.

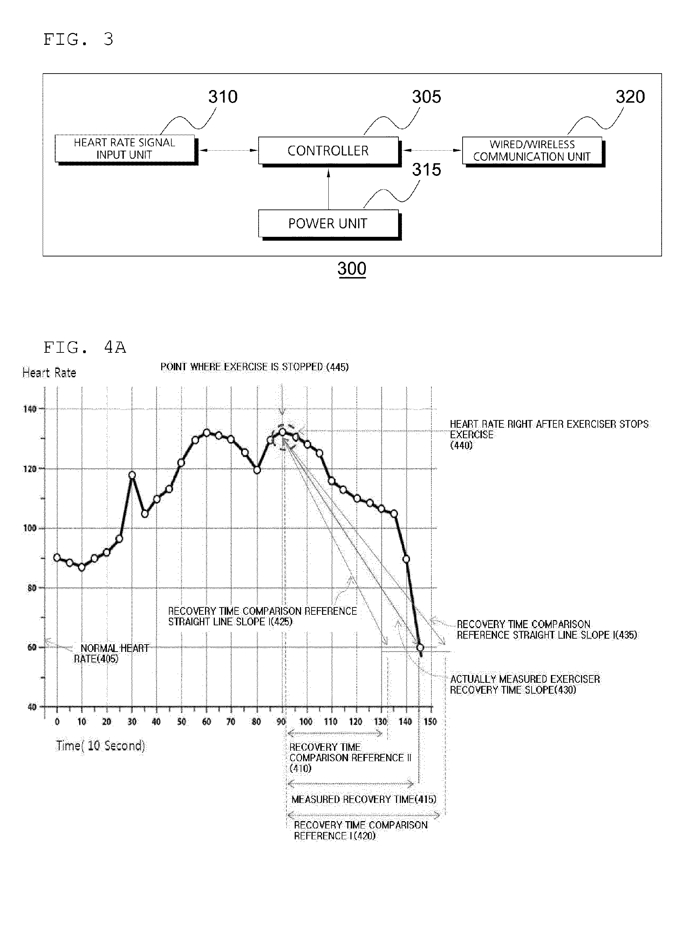

FIG. 3 is a block diagram of a heart rate signal measurement device of a system for diagnosing cardiopulmonary health through a heart rate measurement according to an embodiment of the present invention.

A heart rate signal input unit 310 performs a function of measuring a heart rate signal of a user through the heart rate measurement sensor 205 attached to the temporal artery 100 on a cycle predetermined by a controller 305, and then transmitting the measured heart rate signal to the controller 305.

At this time, if the heart rate measurement sensor 205 is a piezoelectric sensor, the heart rate signal input unit 310 measures the heart rate signal of the user on the cycle determined by the controller 305 and transmits the measured heart rate signal to the controller 305. If the heart rate measurement sensor 205 is a PPG sensor, the heart rate signal input unit 310 supplies light emitting unit output power to a light emitting unit of the PPG sensor. Further, when a strength of the measured heart rate signal is weaker than a predetermined strength, the heart rate signal input unit 310 performs a function of controlling the output of the power supplied to the light emitting unit of the PPG sensor so that a heart rate signal having a strength higher than a specific strength may be measured.

The controller 305 having received the heart rate signal performs a function of transmitting a heart rate signal, in which a motion artifact is removed from the received heart rate signal, to a reception means (a smart phone) through a wired/wireless communication unit 320 and a function of setting the cycle, on which the heart rate signal input unit 310 measures the heart rate signal, in the heart rate input unit 310.

A power unit 310 shown in FIG. 3 supplies power to the controller.

FIG. 4A is an example of a cardiopulmonary health diagnosis graph using a heart rate signal measured before/after exercise.

A general heart rate of a person maintains a range of 60~70 BPM (Beat Per Minute) although there is a difference between individuals, and the heart rate is increased when the person starts exercising.

The increase in the heart rate may vary according to an exercise load, a difference between individuals, a sex, and an age, and the heart rate is increased up to a maximum of 180~220 BPM.

A cardiopulmonary health diagnosis described in the present invention uses a principle in which a heart rate is increased when an exerciser starts exercising, and the increased heart rate 440 is returned to a normal heart rate 405 after a certain time when the exerciser stops exercising. The cardiopulmonary health diagnosis uses a fact that the time required when the increased heart rate 440 of the exerciser is returned to the normal heart rate 405 is different according to the health of cardiopulmonary functions of exercisers although the individual exercisers perform the exercise generating the same exercise load for the same time.

An exerciser having a good cardiopulmonary function among exercisers has a shorter time required when the increased heart rate is returned to the normal heart rate 405 410 and 425, and an exerciser having a bad cardiopulmonary function has a relatively longer time required when the increased heart rate is returned to the normal heart rate 405 420 and 435.

The present invention determines the health of the cardiopulmonary function of the exerciser over time (or a slope of a straight line between two points indicating the heart rate 440 right after the exercise is terminated and the returned normal heart rate 405 required when the increased heart rate 440 due to an exercise by an exerciser is returned to the normal heart rate 405 after the exercise is terminated, hereinafter, referred to as a "a recovery time slope").

According to the present invention, when an exerciser desires to determine whether his/her own health of the cardiopulmonary function is normal, good, or below average by comparing the measured recovery time 415, the exerciser may directly measure heart rate signal data for comparison at the beginning while exercising, and then extract and store the recovery time (or recovery time slope) to use the extracted and stored recovery time. Alternatively, the exerciser may directly identify how much an exercise effect has improved by selecting a stored past recovery time (or recovery time slope) and then comparing the past recovery time with the current recovery time (or recovery time slope) after the exercise. Alternatively, the exerciser may select a normal recovery time (or recovery time slope) for a person of the same age and then compare the normal recovery time with the measured recovery time.

Referring to FIG. 4A, when a recovery time comparison reference straight line slope I 435 and a recovery time comparison reference I 420 are applied, it may be determined that current health of the cardiopulmonary function of an exerciser having an actually measured recovery time 415 and an actually measured exerciser recovery time slope 430 represented by a graph is good. As the health of the cardiopulmonary function of the exerciser becomes better due to a continuous exercise, the measured recovery time 415 may be shorter and the measured exerciser recovery time slope 430 may be smaller.

However, referring to FIG. 4A, when a recovery time comparison reference straight line slope II 425 and a recovery time comparison reference II 410 are applied, it may be determined that that the current health of the cardiopulmonary function of the exerciser having the actually measured recovery time 415 and the actually measured exerciser recovery time slope 430 represented by a graph is below average.

Further, in a method of diagnosing the health of the cardiopulmonary function of the exerciser by using the recovery time comparison reference straight line slope I 435 (or the recovery time comparison reference I 420) and the recovery time comparison reference straight line slope II 425 (or the recovery time comparison reference II 410) together in the use of the graph, it is determined that the health of the cardiopulmonary function of the exerciser is normal when the actually measured exerciser recovery time slope 430 exists between the recovery time comparison reference straight line slope I 435 and the recovery time comparison reference straight line slope II 425, the health of the cardiopulmonary function of the exerciser is below average when the actually measured exerciser recovery time slope 430 exists above the recovery time comparison reference straight line slope I 435 (the actually measured exerciser recovery time slope 430 is smaller than the recovery time comparison reference straight line slope I 435), and the health of the cardiopulmonary function of the exerciser is above average and good when the actually measured exerciser recovery time slope 430 exists below the recovery time comparison reference straight line slope II 425 (the actually measured exerciser recovery time slope 430 is larger than the recovery time comparison reference straight line slope II 425) .

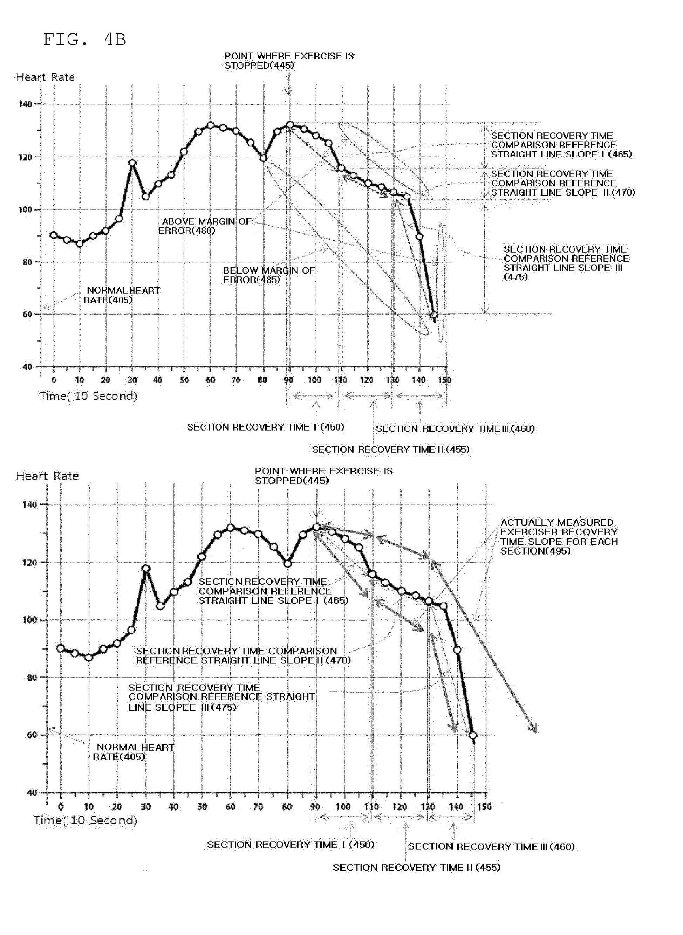

FIG. 4B is an example of a graph for a method of determining the health of the cardiopulmonary function by using section recovery time 450, 455, and 460 and recovery time comparison straight line slopes 465, 470, and 475 for each section.

FIG. 4B is an example of a graph for a method of determining the health of the cardiopulmonary function by using recovery time comparison straight line slopes 465, 470, and 475 for each section with respect to recovery time 450, 455, and 460 for each section preset by a controller or a reception means (smart phone) instead of using the recovery time comparison straight line slopes 425 and 435 and recovery time of entire sections required when the increased heart rate 440 due to the exercise is returned to the normal heart rate 405 after the exercise is terminated, in order to determine more detailed health of the cardiopulmonary function. In the graph of FIG. 4B, sections are preset in the unit of one minute and a performance result is illustrated.

The recovery time comparison straight line slopes 465, 470, and 475 for each section with respect to the recovery time 450, 455, and 460 for each section are configured to be set by the controller or the smart phone such that the slopes of graphs have a margin of error of ±3 to 10 % 490 depending on exercisers. When an actually measured section of an exerciser recovery time slope 495 exists within the margin of error, it is determined that the health of the cardiopulmonary function of the exerciser is good. When an actually measured section of an exerciser recovery time slope 495 exists above the margin of error 480, it is determined that the health of the cardiopulmonary function of the exerciser is below average. When an actually measured section of an exerciser recovery time slope 495 exists below the margin of error 495, it is determined that the health of cardiopulmonary function of the exerciser is above average.

Further, according to the present invention, a step in which the increased heart rate is returned to the normal heart rate 405 after the exercise is set by the controller or the reception means (smart phone) for each section (for example, recovery time required when the increased heart rate is lowered up to 30%, 50%, 70%, and 100% of the normal heart rate of 60~70 BPM at each step), and a recovery time required when the increased heart rate reaches the preset heart rate recovery proportion for each step is measured, so that the health of the cardiopulmonary function of the exerciser may be determined.

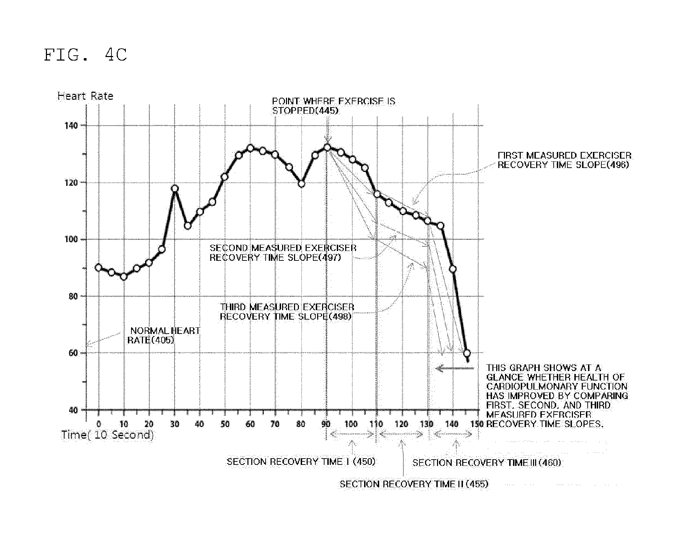

Furthermore, according to the present invention, the health of the cardiopulmonary function of the exerciser may be grasped at the time since the currently measured exerciser recovery time slopes 465, 470, and 475 for each section or the exerciser recovery time slope 430 for the entire section are illustrated together in the heart rate measurement reference graph preset by the exerciser. FIG. 4C is an example of a graph screen for displaying the exerciser recovery time slopes 465, 470, and 475 for each section or the exerciser recovery time slope 430 for the entire section measured during a certain period together to manage a development of an improvement of the health of the cardiopulmonary function according to exercise progress.

The exerciser can compare and analyze an exercise effect at a glance through developments of a first exerciser recovery time slope of entire section 430 or exerciser recovery time slope 465 for each section measured during the certain period, and second and third exerciser recovery time slopes of the entire section 430 or exerciser recovery time slopes 465 for each section.

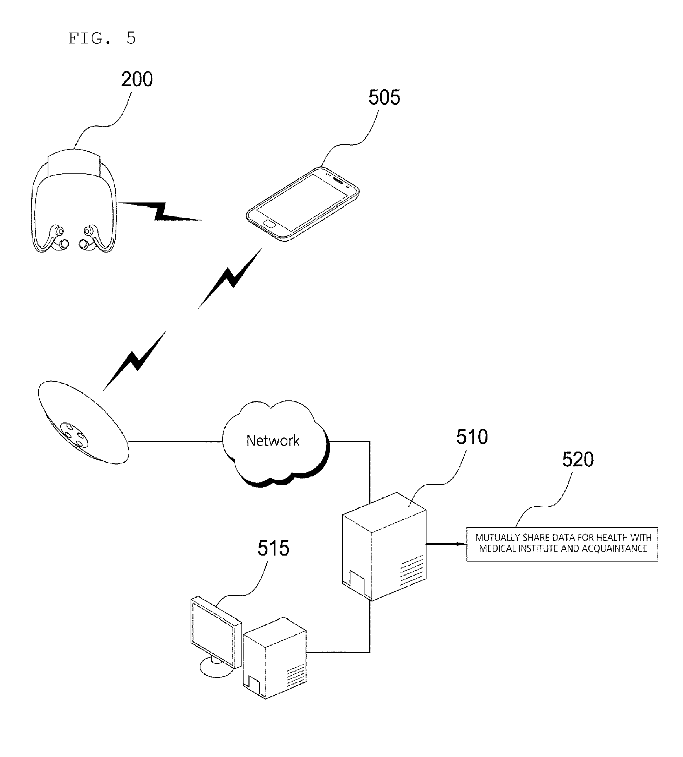

FIG. 5 is a service construction example for diagnosing health of the cardiopulmonary function by using a heart rate signal.

A heart rate signal measured by a heart rate measurement transmission means (headset) 200 is transmitted to a reception means (smart phone) 505 in a wired/wireless communication manner, and the reception means having received the heart rate signal measured by the heart rate measurement transmission means (headset) 100 first-analyzes and displays the measured heart rate signal to enable the exerciser to identify the heart rate signal (in a form such as FIGs. 4A, 4B, and 4C, or in a screen form on which a list and data may be identified).

The exerciser, having identified whether his/her cardiopulmonary health has improved by an exercise result after the exercise, transmits the measured heart rate signal to a management server 510 through wired/wireless communication, so that the exerciser can continuously manage an exercise history and cardiopulmonary health data, and identify his/her own exercise history and development of cardiopulmonary health (in a form such as FIGs. 4A, 4B, and 4C, or in a screen form on which a list and data may be identified) through a remote access anytime and anywhere, or mutually share 520 data for health with an associated medical institution and an acquaintance.

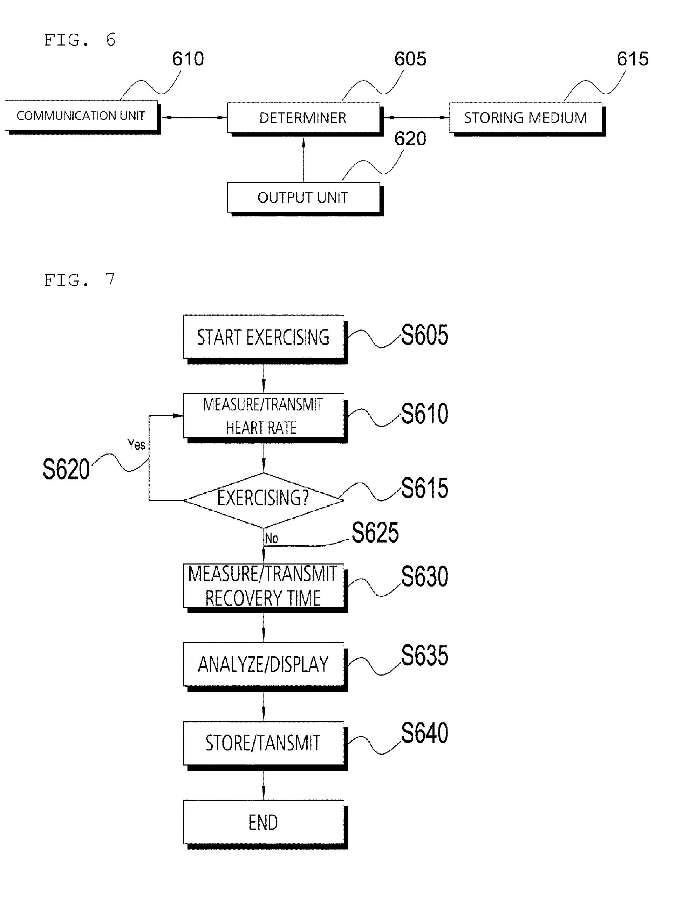

FIG. 6 is an important construction of a smart phone which is a reception means of the present invention.

A communication unit 610 receives a heart rate signal measurement value through the transmission means (headset) 200 having the heart rate measurement sensor 205 for being attached to the temporal artery 100 which is a connecting portion of an ear and a face of a user to sense a heart rate signal of the user. A storing medium 615 performs a function of storing information on an average recovery time (or recovery time slope)- a recovery time required when an increased heart rate of an exerciser due to exercise is returned to a normal heart rate after the exercise (or a recovery time slope which corresponds to a slope of a straight line between two points indicating a heart rate right after the exercise and a normal heart rate returned from the increased heart rate), or further storing information on a past average recovery time (or recovery time slope) of the user.

A determiner 605 analyzes a recovery time required when the increased heart rate of the exerciser due to the exercise is returned to the normal heart rate after the exercise (or a recovery time slope which corresponds to a slope of a straight line between two points indicating a heart rate right after the exercise and a normal heart rate returned from the increased heart rate), and then compares the analyzed recovery time (or recovery time slope) with the average recovery time (or recovery time slope) pre-stored in the storing medium 615, to determine the health of the cardiopulmonary function of the user.

Further, the determiner 605 performs functions of determining whether the health of the cardiopulmonary function of the user is normal, good, or below average through a size of a difference value between the analyzed recovery time (or recovery time slope) and the average recovery time (or recovery time slope) pre-stored in the storing medium 615, and setting a step, in which the increased heart rate after the exercise is returned to the normal heart rate 405, for each section to determine the health of the cardiopulmonary function of the user for each section.

An output unit 620 performs functions of outputting a determination result of the determiner 605 through a screen or a speaker, and outputting a recovery time of the entire section or a recovery time for each section in which the exercise is performed.

FIG. 7 is a flowchart of a method of allowing the cardiopulmonary health of the exerciser to be identified anytime and anywhere by analyzing and displaying the measured heart rate signal.

When the exerciser starts exercising S605, the heart rate signal measurement device 300 installed in the heart rate measurement headset 200 measures a heart rate S610.

When the heart rate signal is measured while the exerciser is exercising S620, the heart rate signal measurement device 300 continuously measures the heart rate of the exerciser and transmits the measured heart rate to the reception means (smart phone) S610. However, during the measurement of the heart rate signal, when the exerciser stops exercising S625, the heart rate signal measurement device 300 measures a heart rate on a preset measurement cycle after the exercise is stopped in order to measure when the increased heart rate due to the exercise is returned to the normal heart rate, and then transmits the measured heart rate to the reception means (smart phone).

The reception means (smart phone) having received the heart rate after the exercise had been stopped generates data for the received heart rate as a graph screen such as FIGs. 4A to 4C to display the data on the screen. Further, the data is displayed together with a comparison heart rate data graph selected before the exercise by the exerciser (the exerciser may select the recovery time (or recovery time slope) extracted and stored after the exerciser directly measures heart rate signal data for comparison at the beginning while exercising, the stored past recovery time (or recovery time slope) after previous exercise, or the normal recovery time (or recovery time slope) of a person of the same age), so that the exerciser can directly recognize the exercise result (whether his/her health of the cardiopulmonary function is good) S635.

The exerciser having identified the exercise result stores the exercise result in the reception means (smart phone) or transmits the exercise result to the management server, so that the exerciser can identify his/her own exercise history and development of the cardiopulmonary health by remotely accessing anytime and anywhere and mutually share data for the health with an associated medical institute and an acquaintance S640.

Through the above construction and operation, a user can identify his/her own heart rate health (cardiopulmonary endurance or electrocardiogram) anytime and anywhere without a separate cost generation by using the heart rate measurement sensor, the reception means, and a recording medium installed in the reception means to analyze the heart rate signal and an effect by which the user can systematically manage an exercise effect after periodically performing the exercise is provided.

It is apparent to those skilled in the art that various changes and modifications may be made therein without departing from the spirit and scope of the invention as defined by the appended claims. Therefore, it is understood that the above description and embodiments have been given for the purpose of illustration and are not intended to limit the invention in any way.

The scope of the present invention shall be construed on the basis of the accompanying claims in such a manner that all of the technical ideas included within the scope equivalent to the claims belong to the present invention, rather than the above detailed description.

## Claims

1. A system for diagnosing cardiopulmonary health by using a heart rate measurement, the system comprising:
a heart rate measurement sensor (205) for sensing a heart rate signal while being in contact with a temporal artery (100) which is a connecting portion of an ear and a face of a user;
a heart rate signal measurement device (300) comprising an input unit (310) for receiving an input of a heart rate signal measurement value from the heart rate measurement sensor (205) being in contact with the temporal artery (100) on a preset period, a wired/wireless communication unit (320) for transmitting the heart rate signal measurement value to a reception means (505) of the user, and a controller (305) for controlling such that the heart rate signal measurement value through the heart rate measurement sensor (205) is transmitted to the reception means (505) of the user on the preset period by controlling the heart rate measurement sensor (205), the input unit (310), and the wired/wireless communication unit (320); and
a transmission means (200) configured to have a body connecting an earphone, the heart rate measurement sensor (205), and the heart rate signal measurement device (300).

2. The system as claimed in claim 1, further comprising:
a storing medium (615) for storing information on an average recovery time (or recovery time slope) in accord with an age and a sex of the user, and a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise;
a communication unit (610) for receiving a heart rate signal measurement value transmitted from the transmission means (200);
a determiner (605) for analyzing a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise, comparing the analyzed recovery time (or recovery time slope) with the average recovery time (or recovery time slope) pre-stored in the storing medium (615), and then determining health of the cardiopulmonary function of the user; and
the reception means (505) configured to have an output unit (620) for outputting a determination result of the determiner (605) through a screen or a speaker.

3. The system as claimed in claim 1 or 2, wherein the controller (305) controls such that the heart rate measurement sensor (205) measures a heart rate signal of the user on a preset period when the heart rate measurement sensor (205) is a piezoelectric sensor, and supplies output power to a light emitting unit of a PPG sensor when the heart rate measurement sensor (205) is the PPG sensor, wherein the controller (305) controls such that a heart rate signal stronger than a predetermined intensity is measured by controlling an output of the power supplied to the light emitting unit of the PPG sensor through a control of a power unit when an intensity of the measured heart rate signal is weaker than a preset intensity.

4. The system as claimed in claim 1 or 2, wherein the controller (305) further removes a motion artifact from a heart rate signal measurement value input through the input unit (310), and sets a period, on which a heart rate is measured, in the input unit (310).

5. The system as claimed in claim 2, wherein the determiner (605) determines whether health of the cardiopulmonary function of the user is normal, good, or below average according to a size of a difference value between the analyzed recovery time (or recovery time slope) and the average recovery time (or recovery time slope) pre-stored in the storing medium (615).

6. The system as claimed in claim 2, wherein the determiner (605) sets a step, in which an increased heart rate after a termination of an exercise is returned to a normal heart rate, for each section, to determine health of the cardiopulmonary function of the user for each section.

7. The system as claimed in claim 2, wherein the output unit (620) outputs a recovery time (or recovery time slope) for an entire section or each section according to an exercise progress.

8. The system as claimed in claim 2, wherein the storing medium (615) further stores information on a past average recovery time (or recovery time slope) of the user, and the determiner (605) compares a recovery time (or recovery time slope) according to a current exercise by the user with the average recovery time (or recovery time slop) pre-stored in the storing medium (615), to determine health of the cardiopulmonary function of the user.

9. The system as claimed in claim 2, wherein the reception means (505) transmits the determination result of the determiner (605) to a third terminal or a server (510) on a communication network.

10. The system as claimed in claim 1, wherein the transmission means (200) corresponds to a headset.

11. The system as claimed in claim 2, wherein the reception means (505) corresponds to a smart phone.

12. The system as claimed in claim 1 or 2, wherein the heart rate measurement sensor (205) is in contact with a temporal artery (100) which is a connecting portion of an ear and a face of a person.

13. A method of diagnosing cardiopulmonary health by using a heart rate measurement, the method comprising:
receiving a heart rate signal measurement value sensed by a heart rate measurement sensor (205) for sensing a heart rate signal while being in contact with a temporal artery (100) which is a connecting portion of an ear and a face of a user from a transmission means (200), by a reception means (505);
generating a recovery time (or recovery time slope which is a slope of a straight line drawn between two points indicating a heart rate right after a termination of an exercise and a normal heart rate returned from the heart rate right after the termination of the exercise) required when an increased heart rate due to the exercise by the user is returned to the normal heart rate after the termination of the exercise, by the reception means (505);
comparing the generated recovery time (or recovery time slope) with a user's age average recovery time (or recovery time slope) or user's previous average recovery time (or recovery time slope) pre-stored in a storing medium (615) to determine health of a cardiopulmonary function of the user, by the reception means (505); and
outputting a comparison result between the generated recovery time (or recovery time slope) and the user's age average recovery time (or recovery time slope) or the user's previous average recovery time (or recovery time slope) pre-stored in the storing medium (615) or a result of the determination on a screen, by the reception means (505).

14. The method as claimed in claim 13, wherein the comparison or the determination result is transmitted to a server (510) on a communication network or a third terminal.

15. A computer readable recording medium for recording a program for executing the method as claimed in claim 13.
